# EUROPEAN PATENT SPECIFICATION

(11) **EP 1 044 678 B1**
(45) Date of publication and mention of the grant of the patent: **12.03.2003**
(21) Application number: 00202069.1
(22) Date of filing: 17.05.1995
(51) Int. Cl.: A61K 9/00

(54) **Lacrimal gland specific emulsions for topical application to ocular tissue**
Tränendrüsenspezifische Emulsionen zur topischen Anwendung an okuläre Gewebe
Emulsions spécifiques de la glande lacrymale pour application topique sur les tissus de l'oeil

(30) Priority: 17.05.1994 US 243279
(43) Date of publication of application: 18.10.2000
(62) Divisional of application: 95921294.5
(73) Proprietor: Allergan Sales, Inc., Irvine, CA 92612 (US)
(72) Inventor: Ding, Shulin, Irvine, CA 92714 (US); Tien, Walter L., Irvine, CA 92714 (US); Olejnik, Orest, Trabucu Canyon, CA 92679 (US)
(74) Representative: Hutchins, Michael Richard

(56) References cited:
- EP-A- 0 028 110
- EP-A- 0 480 690
- EP-A- 0 608 831

## Description

The present invention generally relates to novel ophthalmic aqueous emulsion compositions that can incorporate chemicals (such as cyclosporine) which are poorly soluble in water and is more particularly related to a novel ophthalmic emulsion containing polysorbate 80 with high comfort level and low irritation potential.

Cyclosporins are a group of nonpolar cyclic oligopeptides with known immunosuppressant activity. In addition, as set forth in U.S. Patent No. 4,839,342, cyclosporin (sometimes referred to in the literature as "cyclosporine") has been found as effective in treating immune medicated keratoconjunctivitis sicca (KCS or dry eye disease) in a patient suffering therefrom.

As hereinabove noted, cyclosporin comprises a group of cyclic oligopeptides and the major component thereof is cyclosporin A (C₆₂H₁₁₁N₁₁O₁₂)which has been identified along with several other minor metabolites, cyclosporin B through I. In addition, a number of synthetic analogs have been prepared.

In general, commercially available cyclosporins may contain a mixture of several individual cyclosporins which all share a cyclic peptide structure consisting of eleven amino acid residues with a total molecular weight of about 1,200, but with different substituents or configurations of some of the amino acids.

It should be appreciated that reference to the term "cyclosporin" or "cyclosporins" is used throughout the present specification in order to designate the cyclosporin component in the composition of the present invention.

However, this specific reference is intended to include any individual member of the cyclosporin group as well as admixtures of two or more individual cyclosporins, whether natural or synthetic.

The activity of cyclosporins, as hereinabove noted, is as an immunosuppressant and in the enhancement or restoring of lacrimal gland tearing. This activity can be enhanced if it is possible to enhance the absorption of the cyclosporin in the lacrimal gland.

Unfortunately, the solubility of cyclosporin in water is extremely low and as elaborated in U.S. Patent No. 5,051,402, it has been considered not merely difficult but practically impossible to prepare a pharmaceutical composition containing cyclosporin dissolved in an aqueous medium.

As reported, the solubility of cyclosporin in water is between about 20 µg/ml to 30 µg/ml for cyclosporin A. Hence, heretofore prepared formulations incorporating cyclosporin have been prepared as oily solutions containing ethanol. However, these preparations limit the bioavailability to oral preparations and this is believed to be due to the separation of cyclosporin as a solid immediately after it comes into contact with water, such as in the mouth or eye of a patient.

In the case of injectable preparations of cyclosporin, they first must be diluted with physiological saline before intravenous administration but this is likely to result in the precipitation of cyclosporin and therefore may be considered undesirable for intravenous administration.

Surface active agents such as polyoxyethylated castor oil have been utilized as solubilizers to inject preparations in order to prevent cyclosporin from separating. However, this also may give rise to safety problems (see U.S. Patent No. 5,051,402).

The practical usefulness of cyclosporin would be greatly enhanced if administration thereof could be effective; for example, cyclosporin's effectiveness in the treatment of ocular symptoms of Behcet's Syndrome. However, if it is administered orally for the treatment of these symptoms, the accompanying side effects due to systemic circulation may cause adverse reactions such as hypertrichosis or renal dysfunction.

On the other hand, if oily preparations containing cyclosporin are applied directly to the eyes, irritation or a clouding of visual field may result. This plus the difficulty in formulating cyclosporin limits its use in formulations that would be useful during keratoplasty as well in the treatment of herpetic keratitis and spring catarrh.

Heretofore, as for example in U.S. Patent No. 5,051,402, attempts have been made to dissolve sufficient cyclosporin in an aqueous solvent system so as to reach an effective concentration for treatment. Importantly, this solvent system does not contain any surface active agent such as polyoxyethylated castor oil.

Conceptually, the purpose of dissolving the cyclosporin in an aqueous solvent system is to enable contact with body fluids which would merely constitute dilution of the aqueous solvent system which hopefully would eliminate the immediate precipitation of cyclosporin when contacted with the water content of the body fluids.

For direct use in the eye, cyclosporin has been formulated with a number of pharmaceutically acceptable excipients, for example, animal oil, vegetable oil, an appropriate organic or aqueous solvent, an artificial tear solution, a natural or synthetic polymer or an appropriate membrane.

Specific examples of these pharmaceutically acceptable excipients, which may be used solely or in combination, are olive oil, arachis oil, castor oil, mineral oil, petroleum jelly, dimethyl sulfoxide, chremophor, liposomes, or liposome-like products or a silicone fluid, among others.

In summary, a great deal of effort has been expended in order to prepare a pharmaceutical composition containing cyclosporin dissolved in an aqueous medium or cyclosporin prepared as an oily solution. However, successful formulations have yet to be accomplished as evidenced by the lack of commercial products.

As hereinabove noted, it has been reported that cyclosporin has demonstrated some solubility in oily preparations containing higher fatty acid glycerides such as olive oil, peanut oil, and/or castor oil. These formulations frequently produce an unpleasant sensation when applied to the eye because of stimulation or the viscousness which is characteristic of these oils.

Another drawback of these formulations is that they contain a high concentration of oils, and oils exacerbate the symptoms of certain ocular surface diseases such as dry eyes, indicated by cyclosporin. Therefore, these oily formulations may not be clinically acceptable. Additionally, these formulations often suffer from physical instability due to cyclosporin's propensity to undergo conformational change and crystallize out. The-crystallization problem has been noticed in formulations containing corn oil or medium chain triglycerides. Lastly, these formulations often have a low thermodynamic activity (degree of saturation) of cyclosporin which leads to a poorer drug bioavailability.

It may be possible to minimize the problems related to unpleasant sensation and syndrome exacerbation by reducing the oil content and dispersing the oil phase in water into an emulsion. However, it is not an easy task to formulate an ophthalmic emulsion because one indispensable class of ingredients in an emulsion system is emulsifiers, and the majority of emulsifiers is highly irritating to the eyes.

The present invention is directed to an emulsion system which utilizes higher fatty acid glycerides but in combination with polysorbate 80 which results in an emulsion with a high comfort level and low irritancy suitable for delivery of medications to ocular tissues. The emulsions of the invention contain a cross-linked polyacrylate polyelectrolyte in addition to the polysorbate 80.

EP 0480 690 discloses ophthalmic compositions containing tepoxalin but there would appear to be no disclosure in this document of compositions containing polyelectrolytes.

EP 0 608 831 discloses compositions containing argatroban analogues. Although the compositions can contain polysorbate 80, there is no disclosure of the use of polyelectrolytes.

EP 0028110 discloses autoclavable emulsions containing a polyacrylate as a polyelectrolyte. However, although compositions are disclosed in which polysorbate 80 is present, it is always accompanied by another emulsifier in order to give a desired balance of hydrophilic and lipophilic properties.

### Summary of the Invention

In accordance with the invention, there is provided an ophthalmic aqueous emulsion comprising an emulsifying amount of a higher fatty acid glyceride, a cross-linked polyacrylate polyelectrolyte and polysorbate 80, characterised in that the cross-linked polyacrylate polyelectrolyte and the polysorbate 80 are the only emulsifiers present.

The fatty acid glyceride preferably comprises castor oil.

The emulsions can contain cyclosporin.

Preferably, the weight ratio of the castor oil to the polysorbate 80 is between about 0.3 to about 30 and a weight ratio of the cyclosporin to castor oil is below 0.16. More preferably, the weight ratio of castor oil to polysorbate 80 is between 0.5 and 12.5, and the weight ratio of cyclosporin to castor oil is between 0.12 and 0.02.

When cyclosporin is dissolved in the oil phase in accordance with the present invention, the emulsion is found to be physically stable upon long-term storage. No crystallization of cyclosporin was noticed after nine months at room temperature. Moreover, the cyclosporin emulsion is formulated in such a way that the drug has reasonably high thermodynamic activity, yet without the crystallization problem.

Importantly, an advantage of the composition of the present inventions is that, when formulated with cyclosporin, it provides for enhanced absorption of the cyclosporin in the lacrimal gland of the eye. In this manner, the activity of the cyclosporin in restoring lacrimal gland tearing is increased. That is, since a greater amount of cyclosporin is absorbed into the lacrimal gland, more of the cyclosporin is effective in producing lacrimal gland tearing than heretofore possible.

### BRIEF DESCRIPTION OF THE DRAWINGS

The advantages and features of the present invention will be better understood by the following description when considered in conjunction with the accompanying drawings in which:
Figure 1 is a bar chart of conjunctival concentration of cyclosporin A after a single topical instillation of various formulations in a rabbit eye;
Figure 2 is a bar chart of cornea concentration of cyclosporin A after a single topical instillation of various formulations in a rabbit eye;
Figure 3 is a bar chart of ciliary body concentration of cyclosporin A after a single topical instillation of various formulations in a rabbit eye; and
Figure 4 is a bar chart of lacrimal gland concentration of cyclosporin A after a single topical instillation of various formulations in a rabbit eye.

### DETAILED DESCRIPTION

As hereinabove noted, cyclosporin is available as a mixture in which the principal ingredient is cyclosporin A with significant, but smaller, quantities of other cyclosporins such as cyclosporin B through I. However, as also hereinabove noted, the present invention may be applied to either a pure cyclosporin or to a mixture of individual cyclosporins.

The discovery on which the present invention is founded relates to a combination of a higher fatty acid glyceride and an emulsifier and dispersing agent, polysorbate 80 together with a crosslinked polyacrylate polyelectrolyte. The selection of these components could not have been anticipated on the basis of conventional thinking.

For example, although it is well known that cyclosporin may be used in combination with castor oil, this combination is irritating to sensitive tissues such as the eye. Thus, conventional teaching in the art is away from a formulation which utilizes a higher fatty acid glyceride, such as castor oil, and cyclosporin.

Stated another way, there is no way of deducing that the use of an emulsifier and dispersing agent such as polysorbate 80 will reduce the irritation potential of an emulsion utilizing castor oil. There are no examples of polysorbate in combination with castor oil which, when admixed to cyclosporin, produces an emulsion with a high comfort level and low irritation potential suitable for the delivery of medication to sensitive areas such as ocular tissues.

The emulsions of the present invention, when containing cyclosporin, are advantageous in that they achieve a stable solution state of cyclosporin. This stable solution state is another important performance characteristic differentiating the present invention from the conventional oil systems. Cyclosporin is notorious for its tendency to precipitate out in conventional oil systems in which it is fully dissolved initially.

In accordance with the present invention, the emulsions are further stabilized using a polyelectrolyte, or polyelectrolytes if more than one, from the family of cross-linked polyacrylates, such as carbomers and Pemulen®.

Pemulen® is a polymeric emulsifier having a CTFA name of Acrylates/C10-30 Alkyl Acrylate Cross-Polymer and is discrived in the "Carbomer 1342" monograph in the USPXXII/NPXVII.

In addition, the tonicity of the emulsions can be further adjusted using glycerine, mannitol, or sorbitol if desired. The pH of the emulsions can be adjusted in a conventional manner using sodium hydroxide to a near physiological pH level and while buffering agents are not required, suitable buffers may include phosphates, citrates, acetates and borates.

While the ophthalmic aqueous emulsions in accordance with the present invention can include cyclosporin, other chemicals which are poorly soluble in water such as indomethacin and steroids such as androgens, prednisolone, prednisolone acetate fluorometholone, and dexamethasones, may also be formulated with the emulsions, e.g. containing castor oil and polysorbate 80, resulting in a composition with similar low irritation potential.

The invention is further illustrated by the following examples with all parts and percentages expressed by weight. The cyclosporin used in the examples was supplied by Sandoz.

| Example 1 | | | | | |
|---|---|---|---|---|---|
| | A | B | C | D | E |
| Cyclosporin A | 0.40% | 0.20% | 0.20% | 0.10% | 0.05% |
| Castor oil | 5.00% | 5.00% | 2.50% | 1.25% | 0.625% |
| Polysorbate 80 | 1.00% | 1.00% | 1.00% | 1.00% | 1.00% |
| Pemulen® | 0.05% | 0.05% | 0.05% | 0.05% | 0.05% |
| Glycerine | 2.20% | 2.20% | 2.20% | 2.20% | 2.20% |
| NaOH | qs | qs | qs | qs | qs |
| Purified water | qs | qs | qs | qs | qs |
| pH | 7.2-7.6 | 7.2-7.6 | 7.2-7.6 | 7.2-7.6 | 7.2-7.6 |

| Example 2 | | | | |
|---|---|---|---|---|
| | A | B | C | D |
| Castor oil | 5.00% | 2.50% | 1.25% | 0.625% |
| Polysorbate 80 | 1.00% | 1.00% | 1.00% | 1.00% |
| Pemulen® | 0.05% | 0.05% | 0.05% | 0.05% |
| Glycerine | 2.20% | 2.20% | 2.20% | 2.20% |
| NaOH | qs | qs | qs | qs |
| Purified water | qs | qs | qs | qs |
| pH | 7.2-7.6 | 7.2-7.6 | 7.2-7.6 | 7.2-7.6 |

| Example 3 | |
|---|---|
| | A |
| Castor oil | 2.50% |
| Polysorbate 80 | 0.75% |
| Carbomer® 1382 | 0.05% |
| Glycerine | 2.20% |
| NaOH | qs |
| Purified water | qs |
| pH | 7.2-7.6 |

| Example 4 | |
|---|---|
| | A |
| Castor oil | 5.00% |
| Polysorbate 80 | 0.75% |
| Carbomer® 981 | 0.05% |
| Glycerine | 2.20% |
| NaOH | qs |
| Purified water | qs |
| pH | 7.2-7.6 |

The formulations set forth in Examples 1-4 were made for treatment of keratoconjunctivitis sicca (dry eye) syndrome with Examples 2, 3 and 4 without the active ingredient cyclosporin utilized to determine the toxicity of the emulsified components.

The formulations in Examples 1-4 were applied to rabbit eyes eight times a day for seven days and were found to cause only slight to mild discomfort and slight hyperemia in the rabbit eyes. Slit lamp examination revealed no changes in the surface tissue. In addition, the cyclosporin containing castor oil emulsion, as hereinabove set forth in Examples 1A-1D, was also tested for ocular bioavailability in rabbits; and the therapeutic level of cyclosporin was found in the tissues of interest after dosage. This substantiates that cyclosporin in an ophthalmic delivery system is useful for treating dry eye as set forth in U.S. Patent No. 4,839,342.

In addition, no difference in toxicity was found between formulations with cyclosporin (Examples 1A-1D) and formulations without cyclosporin (Examples 2-4).

The formulations set forth in Examples 1-4 were found to be physically stable upon long term storage. With regard to formulations 1A-1D, no crystallization of cyclosporin was noticed after nine months at room temperature.

Further, other higher fatty acid glycerides such as olive oil, peanut oil and the like may also be utilized with the polysorbate 80 with similar results regarding biotoxicity.

The following examples demonstrate the activity of the composition in accordance with the present invention for enhanced absorption of cyclosporin A in the lacrimal gland.

### Materials

The [Mebmt-³H]-cyclosporin-A (lot #TRQ6553) was prepared by Amersham International (Buckinghamshire, England) with radiochemical purity of -98% (by reversed phase HPLC) and specific activity of 2.6 Ci/mmol (2.16 mCi/mg). The ³H-label is a metabolically stable position as shown by the asterisk. The radiolabeled CsA was supplied as an ethanol solution (1 mCi/ml). All organic solvents used in the procedures described in this study were "HPLC grade". all other chemicals and reagents were analytical grade unless otherwise noted.

The compositions of the six formulations tested are listed in Table A.

**TABLE A**

| Ingredients | Castor Oil | Castor Oil-in-Water Emulsion | Aqueous-α Cyclodextrin | Miglyol Oil-in-Water Emulsion | Polyoxyl 40 | Polyoxyl 40 with Edetate |
|---|---|---|---|---|---|---|
| Cyclosporin-A | 0.20 | 0.20 | 0.10 | 0.20 | 0.05 | 0.05 |
| Cyclodextrin | | | 14 | | | |
| Castor Oil | 99.8 | 1.25 | | | | |
| Miglyol Oil | | | | 20 | | |
| Pluronic L121+P123 | | | | 0.75 | | |
| Tween 80 | | 1.00 | | | | |
| Glycerin | | 2.20 | | 2.20 | | |
| Pemulen® TR-2 | | 0.05 | | | | |
| Carbopol 981 | | | | 0.05 | | |
| Polyoxyl 40 Stearate (mg) | | | | | 20 | 20 |
| HPMC | | | | | 0.3 | 0.3 |
| Butylated Hydroxytoluene | | | | | 0.001 | 0.001 |
| Ethanol(9200 proof) | | | | | | 0.1 |
| Sodium Chloride | | | | | 0.73 | 0.73 |
| Sodium Monophosphate | | | | | 0.2 | 0.2 |
| Disodium Edetate | | | | | | 0.1 |
| Water | | QS | QS | QS | QS | QS |
| Batch Size | 1 g | 5 g | 1 g | 5 g | 1 g | 1 g |

The radiolabeled formulations were formulated to ensure that the radioactivity was homogeneous throughout the vehicle. The expected radioactivity concentrations of the radiolabeled drug formulations were 1-2 mCi/ml. The expected specific activity of radiolabeled cyclosporin A (CsA) formulations was 0.5-2 mCi/mg. All test articles were stored at ambient temperature.

### Analysis of Test Drug Formulations

The test formulations were analyzed in triplicate by HPLC to determine the concentration of CsA and radiochemical purity of the CsA dosing solutions (>93%) before dosing. The radioactive concentrations of the test formulations were quantified by liquid scintillation counting (LSC).

| Chromatographic Conditions | |
|---|---|
| Pump | Beckman Model 126 (Beckman Instruments, San Ramon, CA) |
| | |
| Mobile phase | Acetonitrile: 0.03% H₃PO₄ in water, pH 3 (65:35 v/v) |
| | |
| Flow rate | 1.0 ml/min |
| | |
| Column | Supercosil C8, 7.5 cm × 4.6 mm, 3 µm (Supelco, Bellefonte, PA) superguard LC-8 (Supelco) Column heater (Bio-rad, Richmond, CA) at 60-70°C |
| | |
| Injector | WISP 712B (Waters Associates, Milford, MA) |
| | |
| ¹⁴C detector | Radio Isotope 171 Detector (Beckman Instruments) |
| | |
| Scintillant | Ready Flow III (Beckman Instruments), Flow Rate of 4 ml/min |
| | |
| UV detector | Model 166 (Beckman Instruments), 202 nm |
| | |
| Data processor | Beckman System Gold (Beckman Instruments) |
| | |
| Run Time | 15 min |
| | |
| Retention Time | 6 min (cyclosporin A) |

### Animals

Female New Female New Zealand albino rabbits were obtained and quarantined for at least five days before procedures. Animals were housed in temperature- and humidity-controlled rooms. Food and tap water were provided *ad libitum.* Fifty-eight rabbits (2-3 kg) were selected from the colony to minimize bias. They were individually identified by ear tags and appeared to be healthy.

### Dosing

The animals were divided into six groups of nine rabbits; each group was treated with one of the six CsA formulations. During dosing, the lower eyelid of each rabbit was gently pulled away from the eye and 35 µl of the formulation were administered in the lower conjunctival cul-de-sac of each eye. After dosing, the upper and lower eyelid were handheld closed for ^{~}5 seconds and released. The animals were observed visually for any signs of tearing or ocular discomfort.

### Sampling

Tissues were collected at 20-min., 6-hr. and 24-hr. post-dose for each group. Three rabbits (six eyes) were used at each time point. At the specific sampling times, the animals were euthanized by an intravenous injection of 0.5-1 ml Eutha-6 (Western Supply Co., Arcadia, California) via marginal ear vein. Each eye was then rinsed with normal saline. The aqueous humor (^{~}200 µl) was removed by means of a 0.5 ml tuberculin syringe. The orbital lacrimal gland (^{~}400 mg), upper and lower bulbar conjunctivae (^{~}50 mg each), corneal (^{~}50 mg) and iris-ciliary body (^{~}50 mg) were dissected. The tissues dissected were blotted dry and weighed. Ocular tissue and aqueous humor samples from both eyes were collected from four untreated animals to be used as blank samples.

### Analysis of Radioactivity

An aliquot of aqueous humor (50-175 µl) was counted directly in 10 ml of Ready-Solv HP by LSC. Tissue and blood samples were weighed into combustion cones prior to combustion in a Model 307 Packard Tissue Oxuduzer (Packard Co., Downers Grove, Illinois). After combustion of the tissue samples, ³H₂O was trapped in the Monophase-S solution (Packard) and the radioactivity of the samples was determined by LSC in a Beckman Model 1801 or 3801 scintillation counter (Beckman Instruments, San Ramon, California).

### Data Analyses

Excel software (version 4.0, Microsoft Corp, Redmond, Washington) was used for data analysis. concentrations of total radioactivity in the tissue samples were expressed as dpm/g or dpm/ml and converted to ng equivalents (eq) of CsA/g or ml, using the specific activity of the dosing formulations. Mean, standard deviation (SD) or standard error of the mean (SEM) was calculated according to standard methods. Radioactivity levels were not considered significant unless the dpm was greater than twice that 'of background b=(blanks).

Comparisons of tissue drug concentrations at each time point for the formulations were determined by one-factor ANOVA. All statistical comparisons were made using StatView® (version 1.03, Abacus Concepts, Inc., Berkeley, California). The Fisher and Scheffe F tests were used to determine significant differences between formulations at the 95% level (α = 0.05). The rejection criteria for excluding any outlier data was based on standard outlier tests. No more than one outlier was eliminated from any data set.

### Results and Discussion

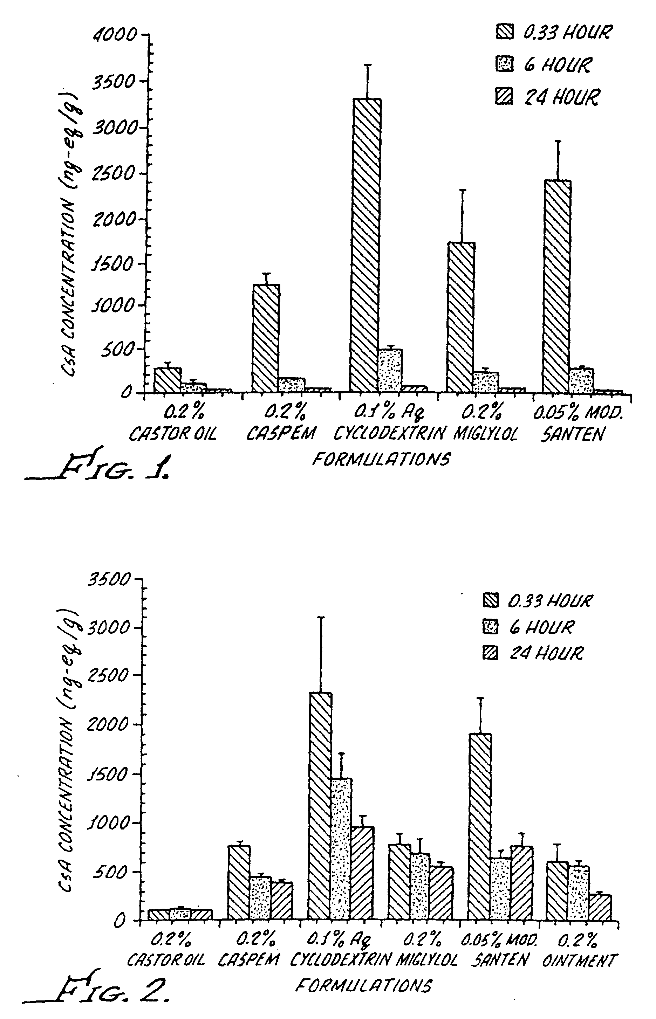

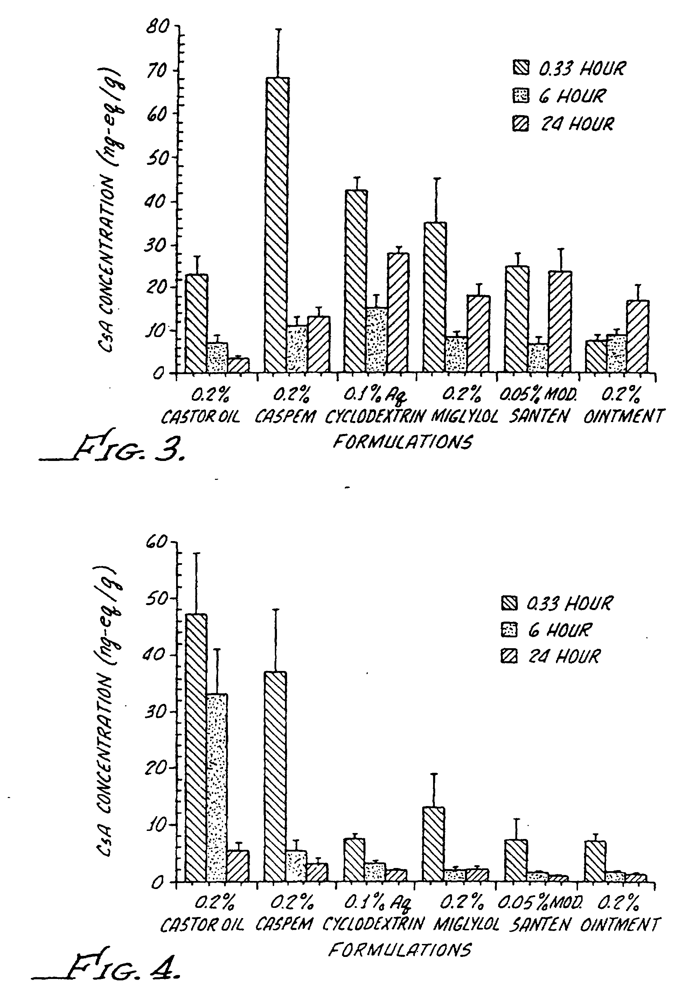

The radioactivity concentrations in ocular tissues at 20 minutes, 6 hours, and 24 hours after a single topical application of various formulations are depicted in Figures 1-4. In general, the concentrations in the ocular tissues were greatest at the earliest time point of 20 minutes as reported in previous single dose studies (2, 3). The radioactivity concentration was highest in the conjunctiva and cornea for each formulation. The relatively low aqueous humor and iris-ciliary body concentrations suggest low intraocular absorption of CsA, consistent with the low CsA corneal permeability of -1.0 × 10⁻⁶ cm/sec (6). The decline of radioactivity concentrations from the cornea was slower than those from the conjunctiva, lacrimal gland, and aqueous humor. The observed blood radioactivity concentrations (<3 ng-eq/ml) were much lower than trough plasma CsA concentrations of 80-250 ng/ml observed after oral dosing to humans (1).

The dependence of CsA corneal and conjunctival penetration on the formulation was interpreted in terms of CsA concentration in formulation and the release rate of CsA from formulation into tear film. The aqueous formulations demonstrated a greater propensity to release CsA for diffusion across the surface tissue epithelia. The 0.2% straight castor oil was formulated below the CsA solubility and therefore the release rate could be hampered by the less than maximal CsA thermodynamic activity (5).

The ocular surface tissues contained a higher fraction of the CsA dose than the other tissues and was used to discriminate among the aqueous, emulsion and the straight castor oil formulations. The polyoxyl 40 formulation produced higher ocular surface tissue concentrations than the emulsions and straight castor oil. The emulsions were also effective in delivery of CsA to the tissues of interest, lacrimal gland, cornea, and conjunctiva. The castor oil emulsion showed higher lacrimal gland concentrations than the modified Santen and the miglyol emulsion. The straight castor oil showed the lowest concentrations in surface ocular tissues. Apparently, the factors influencing CsA penetration into the lacrimal gland and the surface tissues are different.

## Claims

1. An ophthalmic aqueous emulsion comprising an emulsifying amount of a higher fatty acid glyceride, an acrylate/C10-30 alkyl acrylate cross-polymer polyelectrolyte and polysorbate 80, **characterised in that** the acrylate/C10-30 alkyl acrylate cross-polymer polyelectrolyte and the polysorbate 80 are the only emulsifiers present.

2. An ophthalmic aqueous emulsion composition according to claim 1 wherein the higher fatty acid glyceride is selected from olive oil, peanut oil and castor oil.

3. An ophthalmic aqueous emulsion composition according to claim 2 wherein the higher fatty acid glyceride is castor oil.

4. An ophthalmic aqueous emulsion composition according to any one of the preceding claims further comprising a tonicity adjusting agent selected from glycerine, mannitol and sorbitol.

5. An ophthalmic aqueous emulsion composition according to any one of the preceding claims in which the pH of the composition has been adjusted to a near physiological level.

6. An ophthalmic aqueous emulsion composition according to claim 5 wherein the composition contains a buffering agent.

7. An ophthalmic aqueous emulsion composition according to claim 2 and any claim dependent thereon containing a poorly water-soluble pharmaceutically active substance other than cyclosporin.

8. An ophthalmic aqueous emulsion composition according to claim 7 wherein the pharmaceutically active substance is selected from indomethacin and steroids.

9. An ophthalmic aqueous emulsion composition according to claim 8 containing a steroid selected from androgens, prednisolone, prednisolone acetate, fluorometholone and dexamethasone.

10. An ophthalmic aqueous emulsion composition according to claim 1 having a formula as set out in the following table:
| | A | B | C | D |
|---|---|---|---|---|
| castor oil | 5.00% | 2.50% | 1.25% | 0.625% |
| polysorbate 80 | 1.00% | 1.00% | 1.00% | 1.00% |
| crosslinked polyacrylate (Pemulen®) | 0.05% | 0.05% | 0.05% | 0.05% |
| glycerine | 2.20% | 2.20% | 2.20% | 2.20% |
| NaOH | qs | qs | qs | qs |
| purified water | qs | qs | qs | qs |
| pH | 7.2-7.6 | 7.2-7.6 | 7.2-7.6 | 7.2-7.6 |

11. An ophthalmic aqueous emulsion composition according to claim 1 having a formula as set out in the following table:
| | |
|---|---|
| castor oil | 2.50% |
| polysorbate 80 | 0.75% |
| crosslinked polyacrylate (Carbomer® 1382) | 0.05% |
| glycerine | 2.20% |
| NaOH | qs |
| purified water | qs |
| pH | 7.2-7.6 |

12. An ophthalmic aqueous emulsion composition according to claim 1 having a formula as set out in the following table:
| | |
|---|---|
| castor oil | 5.00% |
| polysorbate 80 | 0.75% |
| crosslinked polyacrylate (Carbomer® 981) | 0.05% |
| glycerine | 2.20% |
| NaOH | qs |
| purified water | qs |
| pH | 7.2-7.6 |

## Patentansprüche

1. Wässrige ophthalmische Emulsion mit einer emulgierenden Menge eines höheren Fettsäureglycerids, einem Acrylat/C10-30 Alkylacrylat-Crosspolymer-Polyelektrolyt und Polysorbat 80,
**dadurch gekennzeichnet, dass** der Acrylat/C10-30 Alkylacrylat-Crosspolymer-Polyelektrolyt und das Polysorbat 80 die einzig vorhandenen Emulgatoren sind.

2. Wässrige ophthalmische Emulsionszusammensetzung nach Anspruch 1,
bei der das höhere Fettsäureglycerid aus Olivenöl, Erdnussöl und Rizinusöl ausgewählt ist.

3. Wässrige ophthalmische Emulsionszusammensetzung nach Anspruch 2,
bei der das höhere Fettsäureglycerid Rizinusöl ist.

4. Wässrige ophthalmische Emulsionszusammensetzung nach einem der vorhergehenden Ansprüche, die weiterhin ein Mittel zur Einstellung der Tonizität umfasst, das aus Glycerin, Mannitol und Sorbitol ausgewählt ist.

5. Wässrige ophthalmische Emulsionszusammensetzung nach einem der vorhergehenden Ansprüche, bei der der pH der Zusammensetzung auf ein annähernd physiologisches Niveau eingestellt wurde.

6. Wässrige ophthalmische Emulsionszusammensetzung nach Anspruch 5,
wobei die Zusammensetzung ein Puffermittel enthält.

7. Wässrige ophthalmische Emulsionszusammensetzung nach Anspruch 2 und nach jedem Anspruch, der hiervon abhängig ist, die eine schlecht wasserlösliche pharmazeutisch wirksame Substanz außer Cyclosporin enthält.

8. Wässrige ophthalmische Emulsionszusammensetzung nach Anspruch 7,
wobei die pharmazeutisch wirksame Substanz aus Indomethacin und Steroiden ausgewählt ist.

9. Wässrige ophthalmische Emulsionszusammensetzung nach Anspruch 8, die ein Steroid enthält, das aus Androgenen, Prednisolon, Prednisolonacetat, Fluormetholon und Dexamethason ausgewählt ist.

10. Wässrige ophthalmische Emulsionszusammensetzung nach Anspruch 1, die eine wie in der nachfolgenden Tabelle dargelegte Formel aufweist:
| | A | B | C | D |
|---|---|---|---|---|
| Rizinusöl | 5,00 % | 2,50% | 1, 25 % | 0,625 % |
| Polysorbat 80 | 1,00% | 1,00 % | 1,00% | 1,00% |
| Vernetztes Polyacrylat (Pemulen®) | 0,05 % | 0,05 % | 0,05 % | 0,05 % |
| Glycerin | 2,20 % | 2,20 % | 2,20 % | 2,20 % |
| NaOH | qs | qs | qs | qs |
| Gereinigtes Wasser | qs | qs | qs | qs |
| pH | 7,2-7,6 | 7,2-7,6 | 7,2-7,6 | 7,2-7,6 |

11. Wässrige ophthalmische Emulsionszusammensetzung nach Anspruch 1, die eine wie in der nachfolgenden Tabelle dargelegte Formel aufweist:
| | |
|---|---|
| Rizinusöl | 2,50 % |
| Polysorbat 80 | 0,75 % |
| Vernetztes Polyacrylat (Carbomer® 1382) | 0,05 % |
| Glycerin | 2,20 % |
| NaOH | qs |
| Gereinigtes Wasser | qs |
| pH | 7,2-7,6 |

12. Wässrige ophthalmische Emulsionszusammensetzung nach Anspruch 1 mit einer wie in der nachfolgenden Tabelle dargelegten Formel:
| | |
|---|---|
| Rizinusöl | 5,00 % |
| Polysorbat 80 | 0,75 % |
| Vernetztes Polyacrylat (Carbomer® 981) | 0,05 % |
| Glycerin | 2,20 % |
| NaOH | qs |
| Gereinigtes Wasser | qs |
| pH | 7,2-7,6 |

## Revendications

1. Une émulsion aqueuse ophtalmique comprenant une quantité émulsifiante d'un glycéride d'acide gras supérieur, d'un polymère réticulé de polyélectrolyte d'acrylate/acrylate d'alkyle C₁₀₋₃₀ et du polysorbate 80, **caractérisé en ce que** le polymère réticulé de polyélectrolyte d'acrylate/acrylate d'alkyle C₁₀₋₃₀ et le polysorbate 80 sont les seuls émulsifiants en présence.

2. Une composition d'émulsion aqueuse ophtalmique selon la revendication 1 où le glycéride d'acide gras supérieur est choisi parmi l'huile d'olive, l'huile d'arachide et l'huile de castor.

3. Une composition d'émulsion aqueuse ophtalmique selon la revendication 2 où le glycéride d'acide gras supérieur est de l'huile de castor.

4. Une composition d'émulsion aqueuse ophtalmique selon l'une quelconque des revendications précédentes comprenant en outre un agent d'ajustement de la tonicité choisi parmi la glycérine, le mannitol et le sorbitol.

5. Une composition d'émulsion aqueuse ophtalmique selon l'une quelconque des revendications précédentes où le pH de la composition a été ajusté à un niveau pratiquement physiologique.

6. Une composition d'émulsion aqueuse ophtalmique selon la revendication 5 où la composition contient un agent tampon.

7. Une composition d'émulsion aqueuse ophtalmique selon la revendication 2 et l'une quelconque des revendications dépendantes de celle-ci contenant une substance pharmaceutiquement active faiblement soluble dans l'eau autre que la cyclosporine.

8. Une composition d'émulsion aqueuse ophtalmique selon la revendication 7 dans laquelle la substance pharmaceutiquement active est choisie parmi l'indométhacine et les stéroïdes.

9. Une composition d'émulsion aqueuse ophtalmique selon la revendication 8 contenant un stéroïde choisi parmi les androgènes, la prédnisolone, l'acétate de prédnisolone, le fluorométholone et la dexaméthasone.

10. Une composition d'émulsion aqueuse ophtalmique selon la revendication 1 ayant une formule telle que donnée dans le tableau suivant:
| | A | B | C | D |
|---|---|---|---|---|
| huile de castor | 5,00% | 2,50% | 1,25% | 0,625% |
| polysorbate 80 | 1,00% | 1,00% | 1,00% | 1,00% |
| polyacrylate réticulé (Pemulen®) | 0,05% | 0,05% | 0,05% | 0,05% |
| glycérine | 2,20% | 2,20% | 2,20% | 2,20% |
| NaOH | qs | qs | qs | qs |
| eau purifiée | qs | qs | qs | qs |
| pH | 7,2-7,6 | 7,2-7,6 | 7,2-7,6 | 7,2-7,6 |

11. Une composition d'émulsion aqueuse ophtalmique selon la revendication 1 ayant la formule donnée dans le tableau suivant:
| | |
|---|---|
| huile de castor | 2,50% |
| polysorbate 80 | 0,75% |
| polyacrylate réticulé (Carbomer® 1382) | 0,05% |
| glycérine | 2,20% |
| NaOH | qs |
| eau purifiée | qs |
| pH | 7,2-7,6 |

12. Une composition d'émulsion aqueuse ophtalmique selon la revendication 1 ayant la formule donnée dans le tableau suivant:
| | |
|---|---|
| huile de castor | 5,00% |
| polysorbate 80 | 0,75% |
| polyacrylate réticulé (Carbomer® 1382) | 0,05% |
| glycérine | 2,20% |
| NaOH | qs |
| eau purifiée | qs |
| pH | 7,2-7,6 |
